(19) Europäisches Patentamt
European Patent Office
Office européen des brevets

(11) Numéro de publication: **0 156 740**
**B1**

(12) # FASCICULE DE BREVET EUROPÉEN

(45) Date de publication du fascicule du brevet: **12.10.88**

(51) Int. Cl.⁴: **A 61 F 2/06**, A 61 L 27/00

(21) Numéro de dépôt: **85420025.0**

(22) Date de dépôt: **19.02.85**

(54) **Procédé de fabrication de tubes de collagène notamment de tubes de faibles diamètres et application des tubes obtenus dans le domaine des prothèses vasculaires et des sutures nerveuses.**

(30) Priorité: **21.02.84 FR 8403181**

(43) Date de publication de la demande: **02.10.85 Bulletin 85/40**

(45) Mention de la délivrance du brevet: **12.10.88 Bulletin 88/41**

(84) Etats contractants désignés: **BE CH DE GB IT LI NL SE**

(56) Documents cité:
**WO-A-83/03536**
**FR-A-2 235 133**
**US-A-3 123 482**
**US-A-4 090 878**
**US-A-4 252 759**

(73) Titulaire: **BIOETICA, Société Anonyme, 51 rue Clément Marot, F-69007 Lyon (FR)**

(72) Inventeur: **Huc, Alain, 96 Chemin des Fonds, Sainte- Foy- Lyon (Rhone) (FR)**
Inventeur: **Gimeno, René, Chuyer - Métrieux, Pelussin (Loire) (FR)**

(74) Mandataire: **Maureau, Philippe, Cabinet Germain & Maureau Le Britannia - Tour C 20, bld Eugène Déruelle, F-69003 Lyon (FR)**

EP 0 156 740 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

## Description

La présente invention concerne un procédé de fabrication de tubes de collagène et notamment de tubes de faibles diamètres et l'application des tubes obtenus dans le domaine des prothèses vasculaires et des sutures nerveuses.

Le collagène, qui représente environ 40 % des protéines de l'être vivant, est la substance de soutien des tissus conjonctifs qui lui doivent l'essentiel de leurs fonctions.

On sait que le collagène présente des propriétés mécaniques remarquables, un bon pouvoir hémostatique et qu'il exerce une action dans la croissance cellulaire.

A ces propriétés, il convient d'ajouter deux autres caractéristiques fort intéressantes qui sont sa biocompatibilité et sa biodégradabilite.

La macromolécule de collagène d'une longueur de 3 000 Å et d'une largeur de 15 Å est constituée de trois chaînes peptidiques. Chaque chaîne, d'une masse de 100 000 daltons, est en forme hélicoïdale. Les axes des hélices s'enroulent en hélice autour d'un axe commun à l'intérieur d'une même macromolécule. Entre certaines chaînes peptidiques existent des liaisons réticulaires. L'arrangement ordonné des macromolécules entre elles conduit à la formation de fibres.

Les excellentes propriétés mécaniques du collagène proviennent, en grande partie, de la structure hélicoïdale et des liaisons réticulaires.

Le caractère antigénique du collagène est très faible. De ce fait, le collagène d'origine animale, ne provoque pas de réaction de rejet chez l'être humain, ainsi qu'il ressort d'une étude de TAKEDA,U. et coll. dans le Jal of Toxicology Sciences, Vol 7, suppl. II, 1982 pp 63-91.

Le collagène présente deux avantages importants le rendant adapté à la constitution d'une prothèse vasculaire: d'une part, sa biocompatibilité et d'autre part son aptitude à exercer une action dans la croissance cellulaire. Par contre, dans cette utilisation, le collagène a deux inconvénients: sa biodégradabilité et son pouvoir hémostatique. Cette dernière propriété est particulièrement gênante, car elle risque de provoquer des thromboses, et il ne peut être question de la faire disparaître en détruisant la structure hélicoïdale de la protéine, car alors, la prothèse présenterait de très mauvaises propriétés mécaniques. Enfin, la biodégradabilité du collagène doit être suffisamment faible afin qu'il puisse être réhabité par des cellules avant qu'il soit digéré par les enzymes.

On connaît par ailleurs des prothèses vasculaires synthétiques qui donnent à peu près satisfaction pour des diamètres superieurs à 4 mm; en dessous de cette dimension pourtant, les vaisseaux de remplacement présentent de sérieux inconvénients, en particulier au niveau de la compliance. C'est pourquoi il est apparu important à de nombreux chercheurs de combler cette lacune à l'aide d'un biomatériau à base de collagène dont les qualités biologiques semblaient être d'un grand secours dans ce domaine des prothèses vasculaires.

Dans un premier temps, l'utilisation d'hétérogreffes veineuses s'est révélée la plus aisée et a conduit à des résultats interéssants.

Malheureusement, cette technique est d'un coût élevé et le nombre de prélèvements de veine est limité. C'est pourquoi s'est fait sentir la nécessité de pouvoir préparer des tubes de petits diamètres à base de collagène.

Le brevet roumain RO-A-76 922 décrit un procédé et un dispositif d'obtention d'éléments tubulaires à partir d'un gel de collagène; dans ce procédé, le collagène mis en solution est soumis à une dialyse contre de l'eau distillée additionnée de EDTA puis est transformé en élément tubulaire par une technique d'électrodéposition. Outre le fait qu'il s'agit d'une technique assez lourde à mettre en oeuvre, on peut remarquer que les inventeurs ne donnent aucune indication sur le diamètre minimum qu'il est possible d'obtenir avec ce procédé.

WO-A-8 303 536 décrit un procédé de fabrication de tubes de collagène de faibles diamètres comportant les étapes suivantes:

- extrusion dans une filière cylindrique d'un gel acide aqueux contenant du collagène natif;
- coagulation des parois du tube de collagène sortant de la filière dans un bain de coagulation;
- séchage.

D'autre part, US-A-3 123 482 décrit un procédé de fabrication d'enveloppes de saucisses dans lequel des tubes de diamètre relativement important de matériaux collagéniques sont fabriqués par extrusion, la coagulation étant effectuée sur les parois internes et externes du tube extrudé, le liquide servant à la coagulation des parois internes étant alimenté par un conduit central concentrique équipant la filière.

Dans la communication présentée par CHIGNIER E. HUC A. et ELOY R. au XVIIIème Congrès de la Société Européenne de Recherche Chirurgicale à Stresa en mai 1982, la biocompatibilité du collagène soulignant tout l'intérêt qu'il pourrait y avoir à réaliser des prothèses vasculaires à partir de ces matériaux a été mise en évidence par le test suivant: une pastille constituée d'un collagène très proche de celui des tubes ci-après décrits a été cousue sur une aorte de rat préalablement perforée. Les résultats obtenus ont montré une bonne biocompatibilité du matériau, l'absence de coagulation sanguine au contact du collagène et, enfin, une résistance mécanique suffisante pour résister à la pression sanguine.

Il était donc nécessaire de mettre au point un procédé permettant d'obtenir, à partir de collagène, des tubes de diamètres inférieurs à 4 mm susceptibles d'être appliqués dans le domaine des prothèses vasculaires, et possèdant donc une biodégradabilité suffisamment faible ainsi qu'une thermostabilité satisfaisante.

Une solution à ce problème est apportée par le procédé selon l'invention qui comporte les étapes suivantes: extrusion, dans une filière cylindrique équipée d'un tube central concentrique destiné à recevoir une partie du

bain de coagulation, d'un gel acide aqueux contenant 1,5 % environ de collagène natif, suivie d'une coagulation des parois interne et externe du tube sortant de la filière dans un bain coagulant constitué d'environ 70 % d'acétone pour 30 % d'ammoniaque et d'un séchage, le tube sortant de la filière étant avantageusement déposé sur un support cylindrique femelle de même diamètre que le tube et étant maintenu pendant une durée d'environ deux heures dans le bain coagulant.

Selon un mode de réalisation de l'invention, le séchage du tube est effectué à l'air libre.

Selon un autre mode de réalisation, le séchage du tube est effectué par lyophilisation.

La réticulation du collagène est avantageusement effectuée par déshydratation poussée en étuve à environ 80°C sous un vide d'environ 0,1333 x $10^2$Pa (0,1 mm de mercure) pendant environ 24 heures.

Selon un mode de réalisation préféré de l'invention, le tube de collagène, une fois réticulé, est soumis à un traitement permettant d'introduire des groupes azides dans la molécule sans toutefois occasionner de couplage avec quelque molécule étrangère que ce soit.

On a en effet constaté, par calorimétrie différentielle programmée, que la temperature de début de dénaturation du collagène était de l'ordre de 34°C ce qui signifie que ledit collagène commencera à se dénaturer à une température inférieure à celle de l'organisme, ce qui contribuera à faire perdre au matériau implanté une partie de ses propriétés mécaniques (HUC A. Labo Pharma, 275,307-312-1978)

L'introduction ci-dessus mentionnée de groupements azides dans la molécule de collagène permet d'augmenter la température de début de dénaturation sans pour autant fixer de composés étrangers, souvent nuisibles au comportement biologique du matériau en général et au développement cellulaire en particulier. On augmente ainsi d'une dizaine de degrés Celsius la température de début de dénaturation qui devient alors nettement supérieure à celle de l'organisme.

Il faut toutefois noter que cette stabilisation chimique de la protéine n'est necessaire que lorsque le tube implanté doit être soumis à des contraintes mecaniques importantes ou qu'une faible biodégradabilité est demandée au biomatériau.

Sur un plan plus général, le procédé d'introduction de groupements azides dans la molécule de collagène peut également s'appliquer à des hétérogreffes, dans lesquelles il joue le même rôle que pour les tubes de collagène ce qui peut permettre d'éviter l'emploi de glutaraldéhyde qui est souvent à l'origine de calcification, en particulier dans le cas des valves cardiaques.

La présente invention sera mieux comprise d'ailleurs et ses avantages ressortiront bien de la description qui suit, en référence au dessin schématique annexé dans lequel:

- Figure 1 est une vue en coupe schématique d'une filière destinée à l'extrusion selon l'invention de tubes de diamètres supérieurs à 2 mm;
- Figure 2 est une vue en coupe schématique à un autre mode de réalisation de cette filière plus spécialement adaptée à l'extrusion de tubes de diamètre intérieur inférieur à 2 mm;
- Figure 3 est une vue en coupe très schématique du dispositif servant à la coagulation des tubes;
- Figure 4 est une vue en coupe selon 4-4 de la figure 3.

Sur les figures (2) représente le corps de la filière munie d'un conduit intérieur concentrique (3), le gel de collagène est représenté par (4), le bain de coagulation par (5), (6) représente le bac de coagulation, (7) un support cylindrique femelle et (8) le tube obtenu.

Le procédé de fabrication du tube de collagène selon l'invention comporte essentiellement trois étapes qui vont maintenant être décrites en détail:

- préparation du gel de collagène,
- extrusion et coagulation du tube,
- traitements du tube en vue d'améliorer son comportement "in vivo".

PREPARATION DU GEL DE COLLAGENE

La peau provenant de veau fraîchement abbattu est lavée à l'eau. Les poils et les tissus sous-cutanes sont éliminés mécaniquement à l'aide d'une refendeuse et seul le derme est conservé. Celui-ci est alors haché et broyé. Le broyat obtenu est ensuite lavé par du tampon phosphate à pH 7,8, puis rincé à l'eau permutée. Il est alors placé dans une solution d'acide acétique à pH 3,5. La dilution doit être telle que la concentration en collagène soit de 1,5 %. L'ensemble est homogénéisé par ultra-sons, puis dégazé par agitation sous vide.

FORMATION DU TUBE

Le gel de collagène (4) obtenu dans l'étape précédente est extrudé dans la filière cylindrique (2). L'originalité de cette filière est qu'elle comporte un conduit intérieur (3) concentrique à celui qui amène le gel de collagène (4). Ce conduit (3) sert à introduire le liquide coagulant (5) à l'intérieur du gel de protéine et à donner ainsi au collagène une forme de tube dont le diamètre intérieur sera égal à celui du conduit. Un système d'avancement par vis sans fin (non représenté) permet de déplacer la filière à vitesse constante réglable au-dessus du bain coagulant. Le tube coagulé (8) est déposé sur un support cylindre femelle (7) de même diamètre que le tube placé dans le bac de coagulation (6). Ce système empêche l'écrasement du tube dans le bain coagulant.

Comme on le voit sur la figure 2, le diamètre de la tête de la filière (2) est diminué quand on désire extruder des tubes de 2, voire de 1 mm.

Le tube ainsi formé est laissé au repos pendant environ deux heures dans le bain coagulant. Au bout de ce laps de temps, le tube encore gorgé d'eau est placé sur une tige de polytétrafluoréthylène (P.T.F.E.) (non représentée) de même diamètre et séché à l'air. Après séchage, le tube peut être facilement enlevé de son support.

Le tube coagulé peut être également séché par lyophilisation. Dans ce cas, le tube sec obtenu a une

structure spongieuse et a des parois beaucoup plus épaisses que celui séché à l'air.

Le matériau obtenu est analysé sur trois plans: sur le plan chimique, sur le plan des masses moléculaires et sur le plan de la structure. Le premier groupe d'analyses mettra en évidence le degré de pureté du collagène, le deuxième, l'intégrité des chaînes peptidiques et le troisième, la non destruction de la structure hélidocoïdale de la protéine. Toutes ces analyses sont nécessaires pour être sûr que la protéine possèdera toutes les propriétés du collagène (HUC A. et BARTHOLIN F. revue de l'Institut Pasteur de LYON, 11-N° 2- 179-190 - 1978).

TRAITEMENT DU TUBE

a) Réticulation du collagène

Pour augmenter la résistance de la protéine, le matériau est soumis à une déshydratation poussée en étuve à 80°C sous un vide de 0,1 mm de mercure pendant 24 heures. Un tel traitement crée de nouvelles liaisons réticulaires entre les chaînes peptidiques du collagène et augmente ainsi son insolubilité et diminue sa biodégradabilité.

b) Amélioration de la stabilité du collagène par introduction de groupes azides dans la molécule

Les demandeurs ont, en effet, déterminé qu'il était possible d'amiéliorer cette stabilité en soumettant le collagène à un traitement permettant l'introduction de groupes azides dans la molécule sans pour autant coupler cette molécule à des substances étrangères, comme c'est le cas dans le procédé décrit et revendiqué dans le brevet français FR-A-2 235 133.

On place le matériau pendant huit jours dans une solution méthanolique acide (méthanol pur, concentration en HCl de 0,3 N). Il se produit une méthylation des groupements acides libres du collagène selon le schéma suivant:

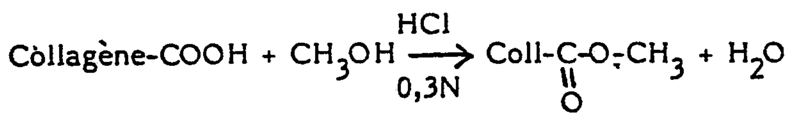


Les groupements acides sont ceux des acides aspartique et glutamique.

Le matériau est ensuite lavé abondamment à l'eau puis place dans une solution aqueuse à 1 % d'hydrazine; les groupements méthylés se transforment en groupements hydrazides selon la réaction:

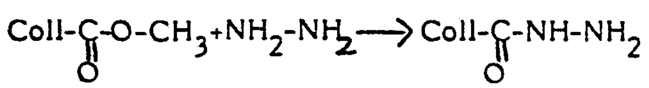

Une durée de quatre à cinq heures est nécessaire pour obtenir le nombre maximum de groupements transformés. Le matériau est alors rincé abondamment à l'eau, puis soumis à l'action d'une solution de nitrite de sodium dans de l'acide chlorhydrique 0,3 N. On obtient alors les groupements azides selon la réaction:

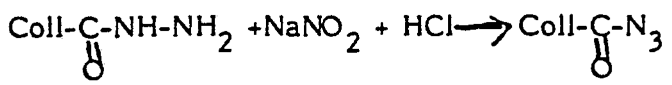

La durée totale de cette réaction est d'environ cinq minutes. Le matériau est ensuite placé pendant deux heures dans du tampon borate pH 9 (acide borique, tétraborate de sodium 0,2 M) puis rincé abondamment à l'eau.

Toutes les étapes du procédé sont réalisées à température ambiante (20° C environ).

Les dosages effectués sur le matériau final montrent qu'il ne contient ni hydrazine, ni nitrite de sodium, ni aucune autre substance étrangère.

La calorimétrie différentielle programmée révèle que la température de dénaturation du collagène est de 10° C plus élevée pour du collagène ainsi traité que pour du collagène brut ou simplement réticulé. Le collagène active est donc plus stable que le collagène reconstitué. La température de dénaturation dépasse alors 37° C, température de l'organisme. Le collagène conservera donc toute sa structure native et, par là, l'essentiel de ses propriétés, en particulier mécaniques; il résistera beaucoup mieux à l'attaque des enzymes protéolytiques.

Un film de collagène ainsi traité a été implanté chez le rat, en situation intrapéritonéale et en situation sous-cutanée. Son comportement a été comparé à celui d'un film de collagène placé dans les mêmes situations.

Les résultats des examens histologiques font ressortir:

- qu'au bout de 21 jours, le film brut a disparu alors que, au bout de 90 jours, le film activé est toujours identifiable;

- que des cellules mésothéliales existent sur les deux implants, mais que leur apparition est retardée sur le matériau activé et qu'elles s'y développent moins bien.

Enfin, une biodégradabilité plus ou moins importante pourra être obtenue en modifiant le temps de contact du collagène avec l'alcool méthylique dans la première étape de la méthode de traitement aux azides. Un temps de contact plus faible entraînera une diminution du nombre de groupements acides bloqués et, par là

même, une protection moindre du collagène. La biodégradabilité du matériau sera donc plus importante que dans le cas du traitement complet.

c) Stérilisation

Le tube est soumis à une dose de rayonnement de 2,5 Mrad. Une telle dose élimine tous les microorganismes, réticule le collagène et diminue son pouvoir hémostatique.

Le procédé selon la présente invention permet donc d'obtenir des tubes simples, tout à fait adaptés à la réalisation de prothèses vasculaires, de diamètre intérieur compris entre 1 et 10 mm.

Les résultats des analyses physico-chimiques et mécaniques des tubes ainsi obtenus sont les suivants:

**ANALYSE CHIMIQUE**

| | |
|---|---|
| - Résidu sec à 105°C | 86,3 |
| - Matières minérales | 0,61 |
| - Acidité (en acide acétique) | 0,33 |
| - Azote | 14,6 |
| - Protéines totales | 79,7 |
| - Hydroxyproline | 9,88 |
| - Collagène | 73,7 |

Les quantités sont données en g pour 100 g de produit brut.

**ANALYSE PHYSIQUE**

| | |
|---|---|
| - Diffraction des rayons X: | Mise en évidence de la structure hélicoïdale |
| -Calorimétrie différentielle programmée: | |
| . température début dénaturation en °C: | 34,3 |
| - température fin dénaturation en °C: | 49,7 |
| Δ H en joule par mg de collagène: | $4,2 \times 10^{-2}$ |
| d'où pourcentage de structure hélicoïdale: | 87 |

**ESSAIS MECANIQUES**

- Contrainte à la rupture $594 \times 10^5$Pa (kg f/cm$^2$: 608) (coefficient variation: 20 %)
- Allongement à la rupture en pour cent: 13 (coefficient variation 55 %)
- Module d'élasticité $11.175 \times 10^5$Pa (kg f/cm$^2$: 11.422) (coefficient variation: 12 %)

On peut noter la valeur élevée de la contrainte à la rupture en kg/cm$^2$.

Quand le tube a été traité par le nouveau procédé de stabilisation décrit ci-avant, les résultats de calorimétrie différentielle programmée sont les suivants:

- Température début dénaturation en °C 47
- Température fin dénaturation en °C 67

soit par rapport au tube non traité une augmentation de 13°C pour la première température et de 8°C pour la deuxième.

Il se peut qu'une simple paroi n'ait pas des propriétés mécaniques suffisantes lorsque la prothèse est soumise à des contraintes mécaniques importantes. Dans ce cas, la prothèse pourra être constituée soit de deux tubes de collagène concentriques collés l'un à l'autre par une colle biologique type Tissucol, soit de deux tubes de collagène entre lesquels se trouve une trame synthétique, par exemple en polyester.

Dans le tableau suivant sont données les compliances respectives d'une veine saphène humaine, d'une prothèse synthétique (Gore-Tex), d'un tube de collagène et de deux tubes de collagène renforcés par une trame en polyester.

Les résultats ont été obtenus dans le laboratoire de Biorhéologie et d'Hydrodynamique Physiologique de l'Université de Paris VII (H. FLAUD).

| Gamme de pression (en Pa) | Compliance en mm3/millibar/mètre 66,66 x $10^2$Pa (50mmHg) | 133 x$10^2$Pa - 266 x $10^2$Pa (100-200 mmHg) |
|---|---|---|
| - Veine saphène humaine | 14,2 | 4,5 |
| - Gore-Tex (diamètre 4 mm) | 2,7 | 2,7 |
| - Collagène (diamètre 4 mm) | 21,5 | 11,6 |
| - Collagène renforcé (diamètre 4 mm) | 5,6 | 5,6 |

Ces résultats montrent que le tube de collagène simple a une compliance plus élevée que la veine saphène et que celle de la prothèse de collagène renforcée par la trame synthétique est plus élevée que celle de la prothèse en Gore Tex.

Les tubes de petits diamètres en particulier peuvent être utilisés pour la réalisation de sutures nerveuses. Les premières expériences faites dans ce domaine ont montré que ce biomatériau permettait de réaliser des jonctions nerveuses dans de bonnes conditions.

**Revendications**

1. Procédé de fabrication de tubes de collagène et notamment de tubes de diamètres inférieurs à 4 mm, comportant les étapes suivantes: extrusion dans une filière cylindrique (2) équipée d'un tube central concentrique (3) destiné à recevoir une partie d'un bain de coagulation (5), d'un gel acide aqueux (4) contenant 1,5 % environ de collagène natif, suivie d'une coagulation des parois interne et externe du tube sortant de la filière dans ledit bain de coagulation (5) constitué d'environ 70 % d'acétone pour 30 % d'ammoniaque, et d'un séchage, le tube sortant de la filière étant dépose sur un support cylindrique femelle (7) de même diamètre et maintenu pendant environ deux heures dans le bain de coagulation.

2. Procédé de fabrication de tubes de collagène selon la revendication 1, caractérisé en ce que le séchage du tube est effectué à l'air libre.

3. Procédé de fabrication de tubes de collagène selon la revendication 1, caractérisé en ce que le séchage du tube est effectué par lyophilisation.

4. Procédé de fabrication de tubes de collagène selon l'une quelconque des revendications 1 à 3, caractérisé en ce que le tube de collagène est soumis, après séchage, à une réticulation par déshydratation poussée en étuve à environ 80° C sous un vide d'environ 0,1333 x $10^2$Pa (0,1 mm de mercure) pendant environ 24 heures.

5. Procédé de fabrication de tubes de collagène selon l'une quelconque des revendications 1 à 3, caractérisé en ce que le tube de collagène, une fois réticulé, est soumis à un traitement permettant d'introduire des groupes azides dans la molécule sans toutefois occasionner le couplage du collagène avec quelque molécule étrangère que ce soit.

6. Procédé de fabrication de tubes de collagène selon la revendication 5, caractérisé en ce que le traitement permettant d'introduire des groupes azides à niveau moléculaire comporte les étapes suivantes:
- methylation des groupements acides libres du collagène,
- transformation des groupements méthylés en groupements hydrazides,
- transformation des groupements hydrazides en groupements azides par action de l'acide nitreux.

7. Procédé de fabrication de tubes de collagène selon la revendication 6, caractérisé en ce que le temps de contact du collagène avec l'alcool méthylique dans l'étape de méthylation est modulé en fonction de la biodégradabilité que l'on désire conférer au collagène.

8. Application du procédé selon l'une quelconque des revendications 1 à 7 à la réalisation de prothèses vasculaires et de sutures nerveuses.

9. Application du procédé selon l'une quelconque des revendications 5 à 7 à des hétéro-greffes.

**Patentansprüche**

1. Verfahren zur Herstellung von Rohren aus Collagen und insbesondere von Rohren mit Durchmessern, die kleiner als 4 mm sind, bestehend aus den folgenden Schritten: Extrusion eines wäßrigen sauren Gels (4), das ungefähr 1,5 % natives Collagen enthält, in einer zylindrischen Ziehdüse (2), die mit einem konzentrischen Zentralrohr (3) ausgestattet ist, das dazu bestimmt ist, einen Teil eines Koagulationsbades (5) aufzunehmen, gefolgt von einer Koagulation der inneren und äußeren Wand des Rohres nach Austritt aus der Ziehdüse in das Koagulationsbad (5), das von ungefähr 70 % Aceton pro 30 % Ammoniak gebildet wird und einer Trocknung, wobei das Rohr (8), das aus der Ziehdüse austritt, auf eine zylindrische Halterung (7) mit dem gleichen Durchmesser aufgelegt wird und während ungefähr 2 Stunden in dem Koagulationsbad gehalten wird.

2. Verfahren zur Herstellung von Collagenrohren nach Anspruch 1, dadurch gekennzeichnet, daß die Trocknung des Rohres im Freien bewirkt wird.

3. Verfahren zur Herstellung von Collagenrohren nach Anspruch 1, dadurch gekennzeichnet, daß die Trocknung des Rohres durch Lyophilisation bewirkt wird.

4. Verfahren zur Herstellung von Collagenrohren nach einem der Ansprüche 1 bis 3, dadurch gekennzeichnet, daß das Collagenrohr nach der Trocknung einer Vernetzung durch hochgradige Dehydratation in der Trockenkammer bei ungefähr 80°C unter einem Vakuum von ungefähr $0{,}1333 \times 10^2$ Pa (0,1 mm Hg) während ungefähr 24 Stunden unterworfen wird.

5. Verfahren zur Herstellung von Collagenrohren nach einem der Ansprüche 1 bis 3, dadurch gekennzeichnet, daß das einmal vernetzte Collagenrohr einer Behandlung unterworfen wird, die es erlaubt, saure Gruppen in das Molekül einzuführen, ohne jedoch die Kupplung des Collagens mit irgendeinem Fremdmolekül zu ermöglichen.

6. Verfahren zur Herstellung von Collagenrohren nach Anspruch 5, dadurch gekennzeichnet, daß die Behandlung, die die Einführung saurer Gruppen auf molekularer Ebene erlaubt, die folgenden Schritte umfaßt:
- Methylierung der sauren freien Gruppierungen des Collagens,
- Umformung der methylierten Gruppierungen in Hydrazidgruppierungen,
- Umformung der Hydrazidgruppierungen in Azidgruppierungen durch die Wirkung von salpetriger Säure.

7. Verfahren zur Herstellung von Collagenrohren nach Ansprüch 6, dadurch gekennzeichnet, daß die Kontaktzeit des Collagens mit Methylalkohol in dem Schritt der Methylierung gesteuert wird als Funktion der biologischen Abbaubarkeit, die man dem Collagen verleihen möchte.

8. Anwendung des Verfahrens nach einem der Ansprüche 1 bis 7 zur Realisierung von Gefäßprothesen und Nervennahtmaterial.

9. Anwendung des Verfahrens nach einem der Ansprüche 5 bis 7 für Heteroplastien.

## Claims

1. A process for the manufacture of tubes of collagen, particularly tubes with diameters less than 4 mm. comprising the following stages: extrusion into a cylindrical nozzle (2) equipped with a central concentric tube (3) intended to receive a portion of a coagulation bath (5), of an acid aqueous gel (4) containing approximately 1.5 % of native collagen, followed by coagulation of the internal and external walls of the tube emerging from the extrusion nozzle into the said coagulation bath (5) that consists of about 70 % of acetone and 30 % of ammonia, and a drying stage, the tube (8) emerging from the extrusion nozzle being laid on a female cylindrical support (7) of the same diameter and retained in the coagulation bath for about two hours.

2. A process for the manufacture of tubes of collagen according to Claim 1, characterized in that the drying of the tube takes place in the open air.

3. A process for the manufacture of tubes of collagen according to Claim 1, characterized in that the drying of the tube takes place by lyophilization (freeze drying).

4. A process for the manufacture of tubes of collagen according to one of Claims 1 to 3, characterized in that the tube of collagen, after drying, is submitted to network formation by forced dehydration in a stove at about 80°C in a vacuum of about $0.1333 \times 10^2$ Pa (0. 1mm Hg) for about 24 hours.

5. A process for the manufacture of tubes of collagen according to one of Claims 1 to 3, characterized in that the collagen tube after network formation is submitted to a treatment enabling azide groups to be introduced into the molecule without however causing the collagen to unite with any foreign molecule of any kind.

6. A process for the manufacture of tubes of collagen according to Claim 5, characterized in that the treatment enabling azide groups to be introduced on a molecular scale comprises the following stages:
- methylation of the free acid groups in the collagen,
- transformation of the methylated groups into hydrazide groups,
- transformation of the hydrazide groups into azide groups by the action of nitrous acid.

7. A process for the manufacture of tubes of collagen according to Claim 6, characterized in that the period of contact with methyl alcohol during the methylation stage is varied according to the degree of biodegradability that it is desired to impart to the collagen.

8. Application of the process according to any of Claims 1 to 7 to produce vascular prostheses and nerve sutures.

9. Application of the process according to any of Claims 5 to 7 to heterografts.

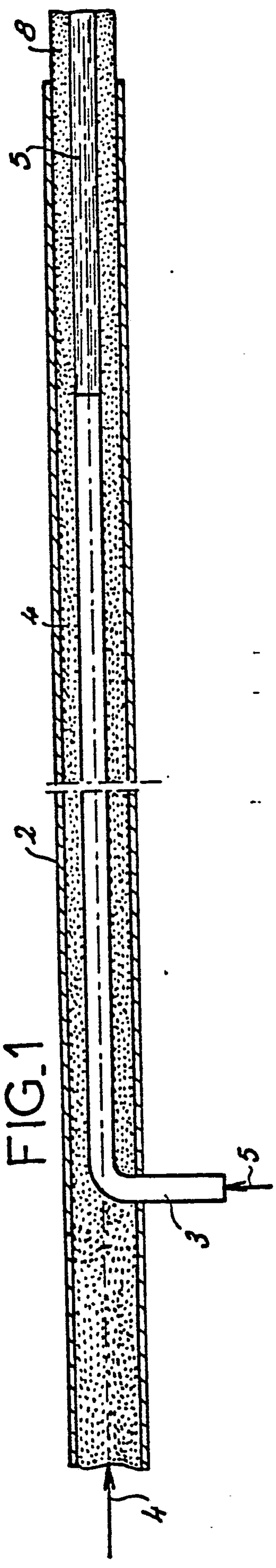

FIG.1

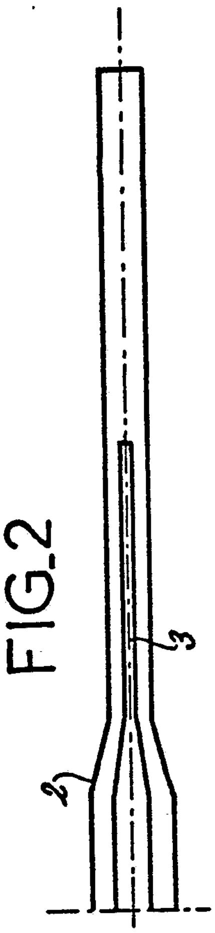

FIG.2

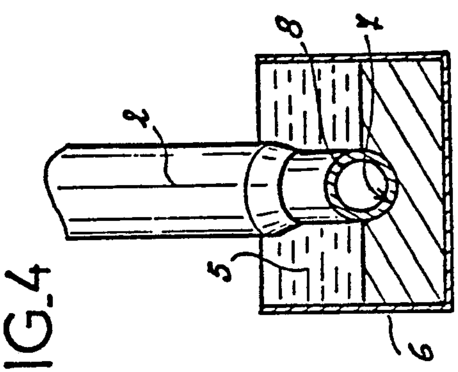

FIG.4

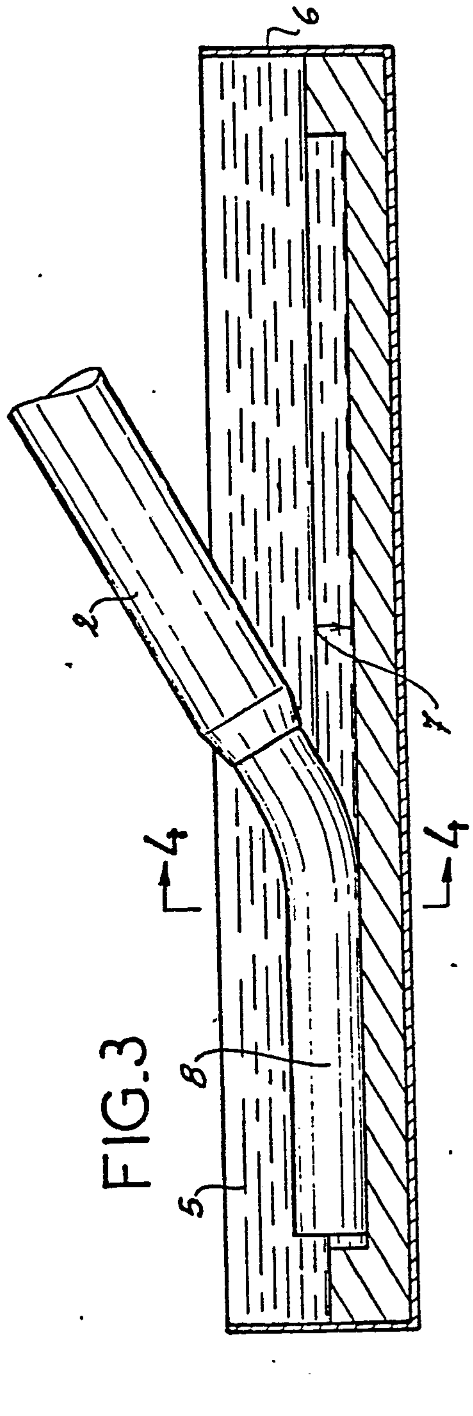

FIG.3